# EUROPEAN PATENT SPECIFICATION

(11) **EP 3 512 876 B1**
(45) Date of publication and mention of the grant of the patent: **27.10.2021**
(21) Application number: 17851361.0
(22) Date of filing: 11.09.2017
(51) Int. Cl.: C07K 14/745, C07K 14/435, A61K 38/36, A61K 38/48, A61K 38/17, C12N 9/64, C12N 5/10, A61P 7/04

(54) **FACTOR VIIA GLYCOFORMS**
FAKTOR VIIA-GLYCOFORMEN
GLYCOFORMES DU FACTEUR VIIA

(30) Priority: 13.09.2016 US 201662393930 P
(43) Date of publication of application: 24.07.2019
(73) Proprietor: Coagulant Therapeutics Corporation, Seoul (KR)
(72) Inventor: FELDMAN, Richard Ira, El Cerrito California 94530 (US)
(74) Representative: Greaves Brewster LLP
(86) International application number: PCT/US2017/050887
(87) International publication number: WO 2018/052827

(56) References cited:
- WO-A1-2013/017555
- WO-A2-2008/083150
- US-A1- 2014 363 419
- APPA RUPA S ET AL: "Investigating clearance mechanisms for recombinant activated factor VII in a perfused liver model", THROMBOSIS AND HAEMOSTASIS, SCHATTAUER GMBH, DE, vol. 104, no. 2, 1 August 2010 (2010-08-01), pages 243-251, XP009156273, ISSN: 0340-6245, DOI: 10.1160/TH09-10-0723
- BOHM, E. ET AL.: 'Differences In N-glycosylation of Recombinant Human Coagulation Factor VII Derived orom BHK, CHO, And HEK293 Cells' BMC BIOTECHNOLOGY vol. 15, no. 87, 18 September 2015, pages 1 - 15, XP055261334
- CHEVREUX, G. ET AL.: 'N-/O-glycosylation analysis of human FVlla produced in the milk of transgenic rabbits' GLYCOBIOLOGY vol. 23, no. 12, 02 October 2013, pages 1531 - 1546, XP055090715

## Description

### FIELD OF THE INVENTION

This disclosure is in the field of biotechnology and concerns the human coagulation protein Factor VII. More particularly, it concerns Factor VII and Factor VIIa glycoforms that have high clearance from a mammalian body

### BACKGROUND OF THE INVENTION

Blood coagulation is a process consisting of a complex interaction of various blood components (or factors) that eventually gives rise to a fibrin clot. Generally, the blood components, which participate in what has been referred to as the coagulation "cascade," are enzymatically inactive proteins (proenzymes or zymogens) that are converted to proteolytic enzymes by the action of an activator (which itself is an activated clotting factor). Coagulation factors that have undergone such a conversion are generally referred to as "active factors" and are designated by the addition of the letter "a" to the name of the coagulation factor (e.g., Factor VIIa).

Initiation of the haemostatic process is mediated by the formation of a complex between tissue factor, which is exposed to the circulating blood following injury to the vessel wall, and Factor VIIa, which is present in the circulation in an amount corresponding to about 1% of the total Factor VII protein mass. This complex is anchored to the tissue factor-bearing cell and converts Factors IX and X to their active forms Factor IXa and Factor Xa on the cell surface. Factor Xa converts prothrombin to thrombin on the tissue factor-bearing cell, which activates Factor VIII, Factor V, Factor XI, and Factor XIII. Furthermore, the limited amount of thrombin formed in this initial step of haemostasis also activates the platelets. Following the action of thrombin on the platelets, the platelets change shape and expose charged phospholipids on their surface. This activated platelet surface forms the template for further Factor X activation and the full thrombin generation. The further Factor X activation on the activated platelet surface occurs via a Factor IXa and Factor VIIIa complex formed on the surface of the activated platelet, and Factor Xa then converts prothrombin into thrombin while still on the surface. Thrombin then converts fibrinogen into fibrin, which is insoluble and which stabilizes the initial platelet plug. This process is localized to the site of the tissue factor exposure thereby minimizing the risk of a systemic activation of the coagulation system. Factor VII and tissue factor have been found to be the main initiators of blood coagulation.

Factor VIIa is also known to activate Factor X directly, without Factor VIIIa or IXa, if given at sufficiently high levels where it acts independent of tissue factor. Therefore, Factor VIIa is used therapeutically, at higher than physiologic levels, to promote coagulation in patients with hemophilia A and B, particularly for patients having inhibitory antibodies to recombinant Factor VIII or Factor IX that limits the effectiveness of replacement therapy with these factors.

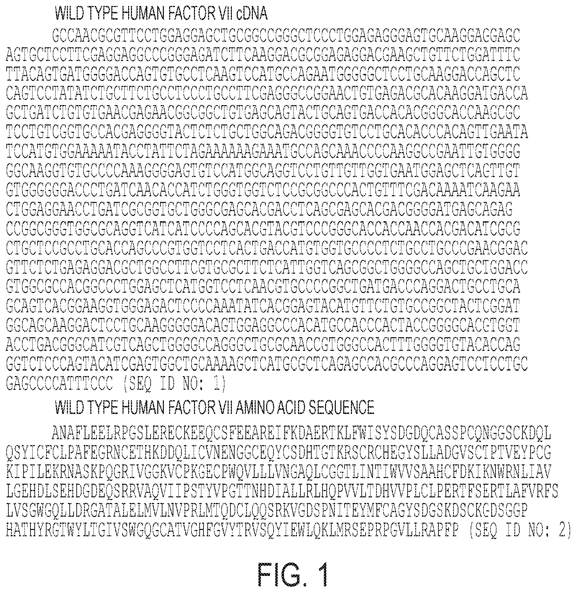

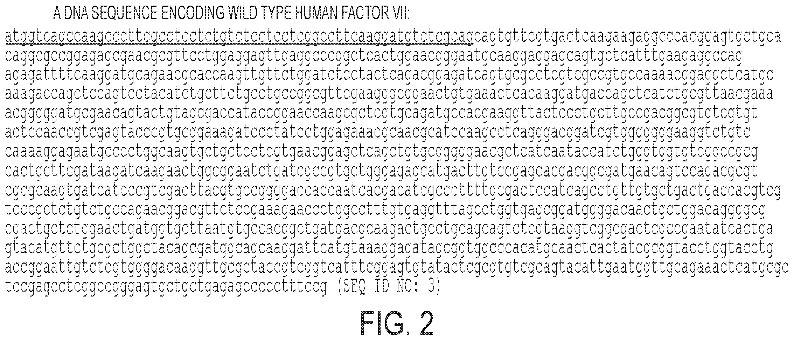

Factor VIIa is produced from its precursor, Factor VII, which is synthesized in the liver and secreted into the blood where it circulates as a single-chain glycoprotein (molecular weight of about 50,000 Da). Wild-type human Factor VII as used herein has the nucleotide sequence and amino acid sequence disclosed in FIGs. 1 and 2 (SEQ ID NOs. 1 and 2, respectively). Wild type Factor VII is cleaved typically between residues 152 and 153 to produce Factor VIIa. A recombinantly produced Factor VIIa polypeptide is commercially sold in the US under the trade name NOVOSEVEN by Novo Nordisk.

The term "Factor VII" means a Factor VII polypeptide in its uncleaved form (the zymogen form). The term "Factor VIIa" means a Factor VIIa polypeptide in its activated two-chain form. When the amino acid sequence of SEQ ID NO: 2 is used herein to describe the amino acid sequence of Factor VIIa it will be understood that one of the chains comprises amino acid residues 1-152, and the other chain amino acid residues 153-406.

Wild type Factor VII is a glycoprotein with N-linked glycosylation sites where sugar residues, called glycans, are attached. Wild type human Factor VII has two N-linked glycans, one at N145 and the other at N322.

In general, the glycans of glycoproteins contribute to the folding of nascent polypeptide chains, e.g. in the endoplasmatic reticulum, and serve to protect the protein moieties from the action of proteases, and serve to modulate biologic activities of a glycoprotein. In contrast to the synthesis of the polypeptide chain of a glycoprotein, which is genetically regulated, the oligosaccharide units or glycans are attached and processed by a series of enzyme reactions. A glycoprotein thus generally appears as a mixture of different glycoforms resulting from varying enzymatic activity.

The oligosaccharide component of the N-glycan structures of glycoproteins can influence therapeutic efficacy, physical stability, resistance to protease attack, pharmacokinetics, interaction with the immune system, and specific biological activity. See, for example, Jenkins et al. (1996) Nature Biotechnol. 14:975-981.

U.S. Patent App. Pub. No. 20140363419 (Bauzon et al.) discloses short acting Factor VII polypeptides useful for treatment of acute bleeding and similar disorders. Bauzon et al. discloses Factor VII variants in which the terminal sialic acid molecules found on the oligosaccharides of wild type or mutant Factor VII are missing, which is referred to as desialylated Factor VII. Bauzon et al. discloses that such desialylated variants have enhanced clearance from the blood and a decrease in the duration of efficacy. Bauzon discloses that desialylated wildtype Factor VIIa is efficacious in coagulation assays but shows no observable systemic coagulation. Due to the great medical need for treatment of hemorrhage, particularly acute hemorrhage, improvements and alternative short-acting Factor VIIa glycoproteins is desired.

U.S. Patent App. Pub. No. 20080058255 (Bolt et al.) discloses glycosylation-disrupted Factor VII variants, such as those having a glycosylation-disrupting substitution at either N145 or N322, or at both N145 and N322. Appa et al, Thrombosis and Haemostasis 104.2/2010, pp. 243-251, reports investigations into clearance mechanisms for recombinant Factor VIIa in which asialo-rFactor VIIa is prepared via enzymatic hydrolysis of recombinant Factor VIIa using neuraminidase agarose. Toso et. al. Blood (abstract), Nov 16, 2000 discloses Factor VIIa having mutations leading to altered glycosylation including desialylation as compared to wild type human Factor VIIa.

Desialylation of glycoproteins is known. For example, U.S. Patent App. No. 20080226681 (Glycotope GmbH) relates to using mutant cells (with a mutation in synthesis of sialic acid) to cause desialylation of proteins. The medium is supplemented with sialic acid precursor. Through the described method, a user may determine the proper degree of sialylation of specific protein to cause the highest activity of the specific protein.

Therapeutic uses for recombinant Factor VIIa are known. However, there is a strong need for a fast acting treatment for acute bleeding conditions, such as postpartum bleed. A fast acting, efficacious Factor VIIa that clears the body rapidly and has few negative coagulation effects on areas outside of the area of trauma is desirable. In particular, there is a need for a fast-acting procoagulant therapeutic with little or no systemic coagulation effects. Improved Factor VIIa glycoforms with Factor VIIa procoagulation activity and rapid and/or high clearance rates are desirable for treatment of acute bleeds.

Bohm, et al., (BMC Biotechnol. 2015 Sep 18;15:87. doi: 10.1186/s12896-015-0205-1. PMID: 26382581; PMCID: PMC4574471) describes differences in N-glycosylation of recombinant human coagulation Factor VII derived from BHK, CHO and HEK293 cells.

Thus, it is an object of the present invention to have Factor VIIa glycoforms and compositions thereof that display pharmacokinetic properties favorable to use for acute bleeding in respect to coagulation activity, half-life, and clearance rate, and have low or no observable systemic coagulation activity.

### SUMMARY

This disclosure concerns a Factor VII or Factor FVIIa glycoprotein having an N-linked glycosylation pattern comprising glycans; wherein the glycosylation pattern comprises:
(1) substantially no glycans with terminal N-acetylneuraminic acid and
(2) a substantial glycan that is a glycan of formula I: (GlcNAc)₄(Man)₃(Gal)₂(Fuc) and
(3) trace amounts or less of one or more glycans selected from the group consisting of a glycan of formula II: (GlcNAc)₅(Man)₃(Gal)(Fuc) and a glycan of formula III: (GlcNAc)₆(Man)₃ (Fuc) and;
(4) a glycan of formula V: (GlcNAc)₆(Gal)₄(Man)₃(Fuc).

The description comprises the above Factor VII or Factor VIIa glycoproteins that further comprise (1) a glycan of formula IV: (GlcNAc)₅(Man)₃(Gal)₃(Fuc). In another embodiment, the Factor VII or Factor VIIa glycoprotein described herein has trace amounts or less of the glycan of formula II. In one further embodiment, the Factor VII or Factor VIIa glycoprotein described herein has trace amounts or less of the glycan of formula III. In a further embodiment, the Factor VII or Factor VIIa glycoprotein described herein comprises an amino acid sequence having three or less mutations relative to SEQ ID NO: 2. In a further embodiment, the Factor VII or Factor VIIa is a Factor VIIa glycoprotein and comprises a heavy chain; wherein the heavy chain has an N-linked glycosylation pattern comprising glycans; and further wherein the glycosylation pattern comprises: (1) substantially no glycans with terminal N-acetylneuraminic acid and (2) a substantial glycan that is a glycan of formula I: (GlcNAc)₄(Man)₃(Gal)₂(Fuc); (3) trace amounts or less of one or more glycans selected from the group consisting of a glycan of formula II: (GlcNAc)₅(Man)₃(Gal)(Fuc) and a glycan of formula III: (GlcNAc)₆(Man)₃ (Fuc); in a further embodiment having trace amounts or less of the glycan of formula III and the glycan of formula I.

In one embodiment, the Factor VII or Factor VIIa glycoprotein as described above and further herein has trace amounts or less of the glycan of formula II and/or the glycan of formula III. The present disclosure also includes methods of making the Factor VII or Factor VIIa glycoproteins described herein, methods for treating a mammal having a disease or a disorder wherein blood clot formation is desirable by administering therapeutically effective amounts of the Factor VII or Factor VIIa glycoproteins described herein, and pharmaceutical formulations of the Factor VII and Factor VIIa glycoproteins described herein in combination with a pharmaceutically acceptable excipient.

### BRIEF DESCRIPTION OF THE DRAWING FIGURES

Figure 1 sets forth the cDNA sequence for human wild type Factor VII (SEQ ID NO: 1) and the human wild type Factor VII amino acid sequence (SEQ ID NO: 2).
Figure 2 sets forth a DNA sequence encoding human wild type Factor VII (SEQ ID NO: 3). The region encoding the signal peptide is underlined.
Figure 3 sets forth a table correlating Roman numerals with glycosylation structures. "Formula" refers to a chemical formula that may be determined by overall glycan mass such as is measured by mass spectroscopy. "Exemplary Structure" shows an example of possible glycan linkages within the scope of the Formula identified by mass. The Exemplary Structures are likely in view of previous published studies of glycans that provided experimentally determined order of the glycan sugars and how they are linked.

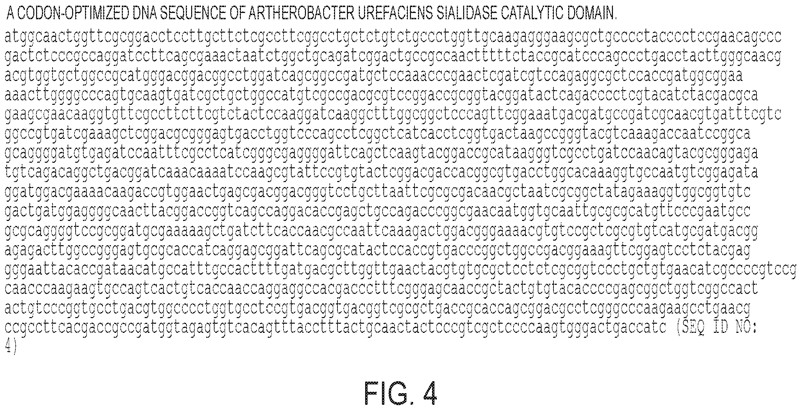

Figure 4 sets forth a codon-optimized DNA sequence of *Artherobacter urefaciens* sialidase catalytic domain (SEQ ID NO: 4).
Figure 5 sets forth the amino acid sequence of *Artherobacter urefaciens* sialidase catalytic domain (SEQ ID NO: 5). A secretion signal sequence from human growth hormone gene (matgsrtslllafgllclpwlqegsa) was added and is underlined.
Figure 6 is a plasmid map for the sialidase expression plasmid pMB279. The base expression vector is the 3 Kb UCOE vector (Millipore) that utilizes a human CMV promoter. The puromycin resistance gene was changed to blasticidin resistance (amino acid sequence of GenBank D83710.1) that was codon optimized for mammalian expression.
Figure 7 is the MALDI (Matrix-assisted laser desorption/ionization mass spectroscopy data from neutral N-glycans for Factor VIIa clones produced in CHO K-1 cells transfected with nucleic acids encoding sialidase and Factor VIIa (subclones 9 and 11), and the MALDI data of the reference preparation, which is NOVOSEVEN recombinant Factor VIIa, referred to as "NOVO 7" herein, desialylated by contact with a sialidase.
Figure 8 is HPLC data analyzing N-glycans for Factor VIIa produced in CHO K-1 cells (subclones 9, 11 and 9 DS) and for the desialylated reference product NOVO 7, after labeling with 2-AA (2-aminobenzoic acid). Subclone 9 DS refers to subclone 9 that has been further purified.

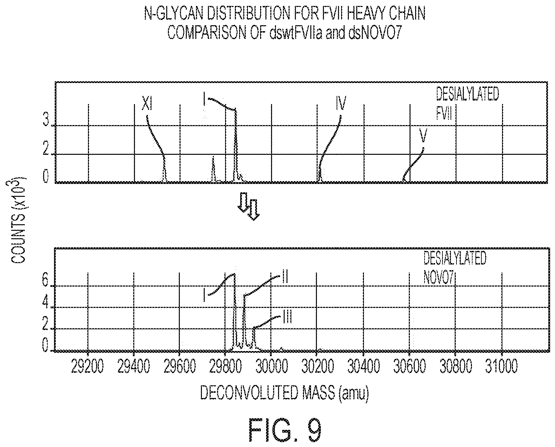

Figure 9 shows mass spectrum for Factor VIIa heavy chain that resolves the glycans present on the molecule. Upper panel shows the N-glycan distribution for Factor VIIa heavy chain in Factor VIIa of the present invention that was produced in CHO cells also expressing sialidase. Figure 9 (lower panel) shows the N-glycan distribution for Factor VIIa heavy chain for NOVOSEVEN recombinant Factor VIIa desialylated by contact with a sialidase. The downward arrows mark certain differences between the glycans on desialylated Factor VIIa according to the present disclosure and glycans on desialylated NOVOSEVEN Factor VIIa.

### DETAILED DESCRIPTION OF THE INVENTION

Factor VII and Factor VIIa glycoproteins having specific N-linked glycosylation patterns, methods of their manufacture and use, pharmaceutical compositions comprising them, methods of treatment of disorders where blood clotting is desired, and cell lines to produce them are described herein. The Factor VII and Factor VIIa glycoproteins have reduced sialic acid content with reference to wild type human Factor VII, and rapid clearance.

Applicants have discovered recombinant Factor VII and Factor VIIa glycoproteins that are rapidly cleared from the human body and whose pharmacokinetic properties make them suitable for therapeutic use in acute bleeding disorders. The glycoproteins' rapid clearing reduces unnecessary exposure to a pro-thrombotic molecule after the bleeding is resolved. The glycoprotein of the present disclosure has a distinct glycosylation profile when compared to NOVOSEVEN recombinant Factor VIIa (Novo Nordisk) and when compared to NOVOSEVEN recombinant Factor VIIa that has been desialylated by contact with sialidase according to prior teachings.

### Definitions

Unless defined otherwise, all technical and scientific terms used herein generally have the same meaning as commonly understood by one of ordinary skill in the art to which this disclosure belongs. Generally, the nomenclature used herein and the laboratory procedures in cell culture, molecular genetics, organic chemistry, and nucleic acid chemistry and hybridization are those well-known and commonly employed in the art. Standard techniques are used for nucleic acid and polypeptide synthesis. The nomenclature used herein and the laboratory procedures in analytical chemistry and organic synthesis described below are those well-known and commonly employed in the art. Standard techniques, or modifications thereof, are used for chemical syntheses and chemical analyses. Procedures used for genetic engineering are well known and can be found, for example, in Sambrook et al., Molecular Cloning: A Laboratory Manual, Cold Spring Harbor, N.Y.

The term "sialic acid" or "sialyl" refers to any member of a family of nine-carbon carboxylated sugars. The most common member of the sialic acid family is N-acetyl-neuraminic acid (2-keto-5-acetamido-3,5-dideoxy-D-glycero-D-galactononulopyranos-1-onic acid (often abbreviated as Neu5Ac, NeuAc, or NANA)).

The terms "polypeptide" and "protein" are used interchangeably herein and refer to a polymer in which the monomers are amino acids and are joined together through amide bonds. As used herein, "polypeptide" and "protein" refer to both glycosylated and unglycosylated polypeptides and proteins, respectively.

The term "glycoprotein" refers to a protein having at least one glycan attached. It is understood that glycans are created and formed through enzymatic processes in the cell.

The term "glycoform" as used herein refers to a glycoprotein containing a particular carbohydrate structure or structures. A glycoprotein having more than one glycosylation site can have the same glycan species attached to each glycosylation site, or can have different glycan species attached to different glycosylation sites. In this manner, different patterns of glycan attachment yield different glycoforms of a glycoprotein.

The terms "glycosylation pattern" and "glycosylation profile" are used interchangeably herein and mean the characteristic "fingerprint" of the representative N-glycan species that have been released from a glycoprotein composition or glycoprotein product, either enzymatically or chemically, and then analyzed for their carbohydrate structure, for example, using LC-HPLC, or MALDI-TOF MS, and the like. See, for example, the review in Current Analytical Chemistry, Vol. 1, No. 1 (2005), pp. 28-57.

The term "trace amount" of a glycan means that very low levels of that glycan are present in comparison with the amount of other glycans in the glycoprotein. Without limitation, a "trace amount or less" of a glycan means that a MALDI-TOF MS, LC-HPLC or similar analytical test shows a glycosylation profile in which the glycan is present at less than 10%, preferably less than 5%, preferably less than 4%, less than 3%, less than 2%, less than 1%, and less than 0.5% or less than 0.1% of the total amount of N-glycan species appearing in the profile, or that is not detectable in the analytical test.

The term "significant glycan" or "substantial glycan" refers to a glycan that is observed under MALDI-TOF MS, LC-HPLC or similar analytical tests to be present in the glycosylation profile as being more than 10%, more than 20%, more than 25%, or more than 30% of the total amount of N-glycan species appearing in the profile. A quantitative determination of glycans may be made, for example, by LC-MS analysis in which the area under the curve (AUC) of the peaks is compared to determine the relative quantity of each glycan in a protein batch. Alternatively, the "significant glycan" or "substantial glycan" may be present in the glycosylation profile as 10%, 11%, 12%, 13%, 14%, 15%, 16%, 17%, 18%, 19%, 20%, 21%, 22%, 23%, 24%, 25%, 26%, 27%, 28%, 29%, 30%; 31%, 32%, 33%, 34%, 35% or more of the total amount of N-glycan species appearing in the glycosylation profile for the product.

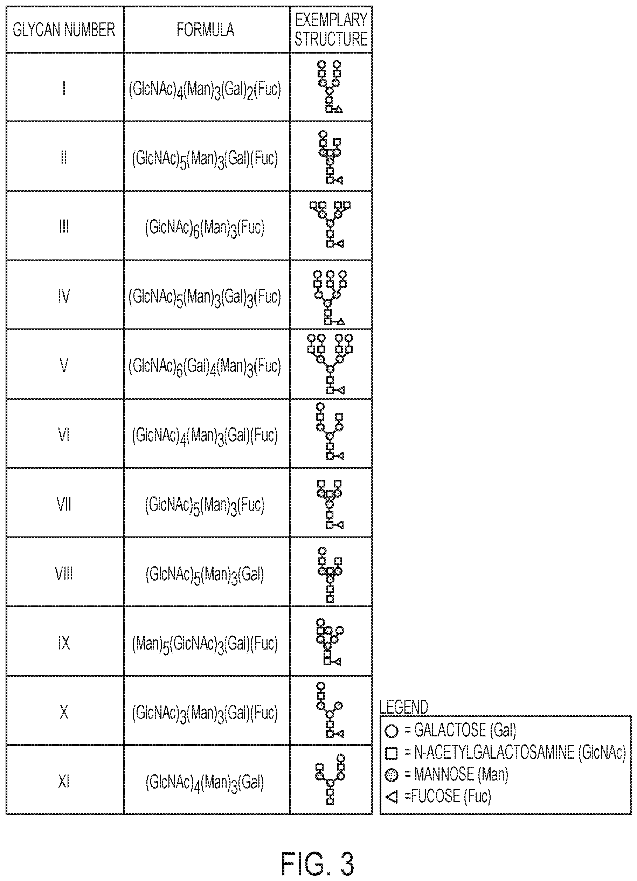

"N-glycan," "N-linked glycan," and "glycan" are used interchangeably and refer to an N-linked oligosaccharide, e.g., one that is or was attached by an N-acetylglucosamine (GlcNAc) residue linked to the amide nitrogen of an asparagine residue in a protein. The predominant sugars found on glycoproteins are glucose, galactose (Gal), mannose (Man), fucose (Fuc), N-acetylgalactosamine (GalNAc), N-acetylglucosamine (GlcNAc), and sialic acid (e.g., N-acetyl-neuraminic acid (NeuAc)). A "*" at the end of a glycan represents the reducing end of the sugar chain that binds to asparagine. An N-linked glycan can be shown by symbolic depiction such as those in Figure 3. The symbols disclosed in Figure 3 are used in other figures and have the same meaning throughout their use herein.

By "oligomannosidic core structure" or "trimannose core structure" of a complex N-glycan is intended the core structure that comprises three mannose (Man) and two N-acetylglucosamine (GlcNAc) monosaccharide residues that are attached to the asparagine residue of the glycoprotein. Subsequent glycosylation steps yield the final complex N-glycan structure.

The N-glycans attached to glycoproteins differ with respect to the number of branches (antennae) comprising peripheral sugars (e.g., GlcNAc, galactose, fucose, and sialic acid) that are added to the trimannose core structure. N-glycans are commonly classified according to their branched constituents (e.g., complex, high mannose, or hybrid). A "complex" type N-glycan typically has at least one GlcNAc attached to the 1,3 mannose arm and at least one GlcNAc attached to the 1,6 mannose arm of a "trimannose" core. Where one GlcNAc is attached to each mannose arm, the species of N-linked glycan is denoted as "GlcNAc ₂Man₃GlcNAc₂." Where only one GlcNac is attached, the N-glycan species is denoted as "GlcNAciMan ₃GlcNAc₂", wherein the GlcNac is attached to either the 1,3 mannose arm (denoted "MGn") or the 1,6 mannose arm (denoted "GnM"). Complex N-glycans may also have galactose ("Gal") or N-acetylgalactosamine ("GalNAc") sugar residues that are optionally modified with sialic acid or derivatives (e.g., "NeuAc," where "Neu" refers to neuraminic acid and "Ac" refers to acetyl). Where a galactose sugar residue is attached to each GlcNAc on each mannose arm, the species of N-linked glycan is denoted as "Gal₂GlcNAc₂Man₃GlcNAc₂." Complex N-glycans may also have intrachain substitutions comprising "bisecting" GlcNAc and core fucose ("Fuc"). Complex N-glycans may also have multiple antennae on the "trimannose core," often referred to as "multiple antennary glycans." A "high mannose" type N-glycan has five or more mannose residues. A "hybrid" N-glycan has at least one GlcNAc on the terminal of the 1,3 mannose arm of the trimannose core and zero or more mannoses on the 1,6 mannose arm of the trimannose core.

Many molecules of the present disclosure have very high clearance from circulation in a human. The clearance may be measured by means known to those of ordinary skill in the art, such as by measuring the terminal half-life, which is measured after any initial distribution of the molecule occurs.

Half-life can be determined in test animals, for example, by administering a dose of about 25-250 microgram/kg of the preparation; obtaining plasma samples at predetermined times after administration; and determining the content of the Factor VIIa polypeptide in the samples using one or more of a clotting assay (or any bioassay), an immunoassay, or an equivalent. The data can be displayed graphically and then the bioavailability will be determined as the area under the curve. In certain examples, rat or murine models are used for half-life measurements. Relative bioavailability of a Factor VIIa polypeptide or composition thereof refers to the ratio of the area under the curve of the short-acting Factor VIIa polypeptide to that of wild-type Factor VIIa or another appropriate comparator polypeptide or protein.

The glycoproteins of the present disclosure may be produced in host cells. A cell may be "derived from" a host cell when it is obtained from the host cell by any of the normal growth and culture techniques known to those of ordinary skill in the art, such as normal mitotic cell division, cloning such as single cell dilution cloning, etc. "Derived from" a host cell also includes recombinant modifications made to the host cell that do not affect the primary function of expressing Factor VII and sialidase. A heterologous protein is a protein that a cell has been engineered to produce.

### Preferred Embodiments

In one embodiment, the Factor VII or VIIa glycoprotein comprises one or two, preferably two, N-linked glycans and has a glycosylation pattern characterized by reduced sialylation compared to wild type human Factor VII or VIIa. Preferably the moles of conjugated sialic acid per mole of N-linked glycan in the glycoprotein is less than 0.05, less than 0.1, less than 1.0, less than 2.0, less than 3.0, less than 4.0, less than 5.0 or less than 6.0, or the ratio of moles of conjugated sialic acid per mole of N-linked glycan is within a range selected from the group consisting of (1) from 0 to 8; (2) from 0 to 7; (3) from 0 to 6; (4) from 0 to 5; (5) from 0 to 4; (6) from 0 to 3; (7) from 0 to 2; (8) from 0 to 1 and (9) from 0 to 0.5, or ratios of from 1 to 8, 1 to 7, 1 to 6, 1 to 5, 1 to 4, 1 to 3, 1 to 2, 2 to 8, 2 to 7, 2 to 6, 2 to 5, 2 to 4, 2 to 3, 3 to 8, 3 to 7, 3 to 6, 3 to 5, 3 to 4, 4 to 8, 4 to 7, 4 to 6, 4 to 5, and 0.1 to 1. The ratio is a measurement of the moles of sialic acid bound to a glycoprotein relative to the number of glycans on the glycoprotein. Most preferably, the glycoprotein has one or two N-linked glycans and trace amounts or less of sialic acid covalently attached to these glycans. Still more preferably, the glycoprotein has substantially no glycans with terminal N-acetylneuraminic acid, and has two N-linked glycans. The number of glycans refers to the number of sugar moieties attached to an N-linked glycan in the glycoprotein, where one N-linked glycosylation site can support only one glycan as defined herein for purposes of this ratio.

The ratio is determined using a sialic acid fluorescence labeling kit such as that sold by Takara Bio Inc. (cat. #4400). Such a sialic acid fluorescence labeling kit includes a step for the release of sialic acid from the bound glycoprotein, such as by partial acid hydrolysis or by use of sialidase, such as Arthrobacter ureafaciens sialidase. The free sialic acids are then labeled with a fluorophore such as 1,2-diamino-4,5-methyleneoxybenzene ("DMB"). The labeled sialic acids are then quantitatively measured using HPLC and comparing peak heights to a calibration curve. Thus, the ratio measured is a ratio of moles of sialic acid per mole of glycan released from all the Factor VII or Factor VIIa glycoproteins of the composition.

By having reduced sialylation, the Factor VII and VIIa glycoproteins have a shorter terminal half-life than wild type Factor VII or VIIa.

In one series of embodiments, the compositions of Factor VII or Factor VIIa glycoproteins or the isolated polypeptides themselves have a terminal half-life as measured in human or mammalian plasma, for example murine or rat plasma, of less than 2 hours, less than 1.5 hours, less than 1 hour, less than 0.75 hour, less than 0.5 hour, less than 0.25 hour, less than 0.1 hour, or so short that it cannot reasonably be measured.

The Factor VII or Factor VIIa glycoform preferably has an N-linked glycosylation pattern that has substantially reduced sialylation as disclosed above, such as substantially no glycans with a terminal N-acetylneuraminic acid, and has a substantial glycan that is a glycan of formula I and trace amounts or less of the glycan of formula II and/or of the glycan of formula III. In another embodiment, the Factor VII or Factor VIIa glycoform has trace amounts or less of glycans of formula III, in another embodiment has trace amounts or less of glycans of formulae II and III, and in another embodiment has glycans of formula I and trace amounts or less of glycans of formulae II and III. In another embodiment, the Factor VII or Factor VIIa glycoform has an N-linked glycosylation pattern that has substantially reduced sialylation as disclosed above, such as substantially no glycans with a terminal N-acetylneuraminic acid, and has glycans of formula IV, in another embodiment has glycans of formula IV and formula V, in another embodiment has glycans of formulae IV, V and XI, and in another embodiment has glycans of formulae IV, V, XI, and I. In one embodiment, the Factor VII or Factor VIIa glycoform has an N-linked glycosylation pattern that has substantially reduced sialylation as disclosed above, such as substantially no glycans with a terminal N-acetylneuraminic acid, and has trace amounts or less of glycans of formula II and/or of formula III.

The glycan patterns as described herein are described by mass-derived formulae that may be generic to encompass more than one structure having the same mass but different linkages between the glycan. The "Exemplary Structure" column in Figure 3 shows possible glycan linkages for the structures of each of formulae I-XI.

Using nomenclature that identifies specific glycan linkages, and is consistent with the glycan mass data described herein, one embodiment of the invention is a Factor VII or Factor VIIa glycoprotein having an N-linked glycosylation pattern comprising glycans; wherein the glycosylation pattern comprises:
(1) substantially no glycans with terminal N-acetylneuraminic acid and
(2) a substantial glycan that is a glycan of the following formula Ia 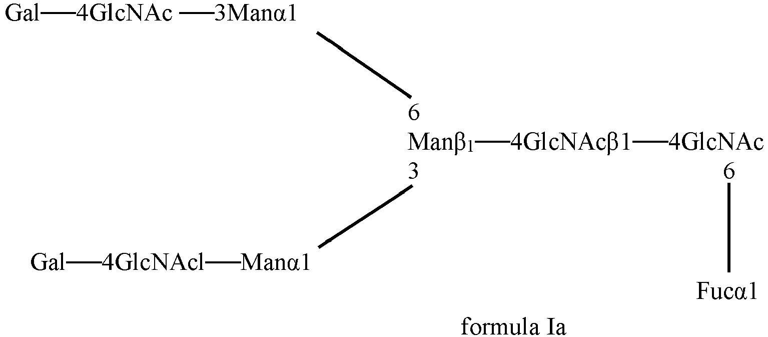 and
(3) trace amounts or less of one or more glycans selected from the group consisting of a glycan of formula IIa: 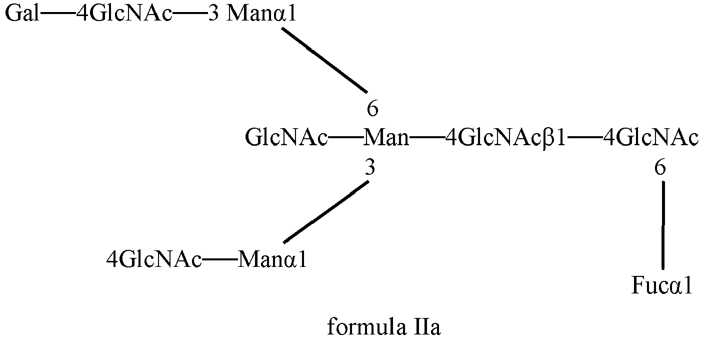 and a glycan of formula IIIa: 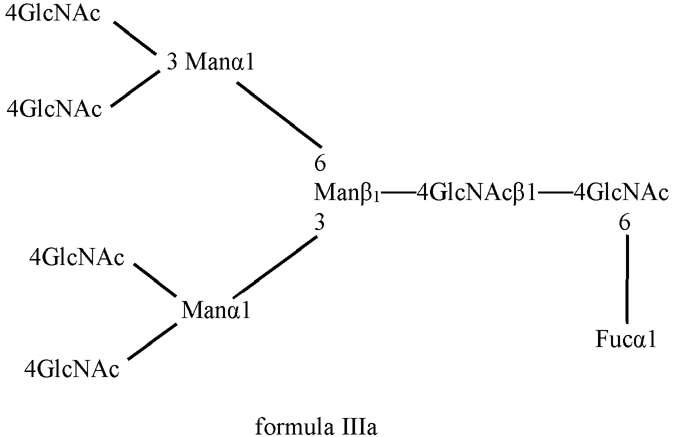 and a glycan of formula Va

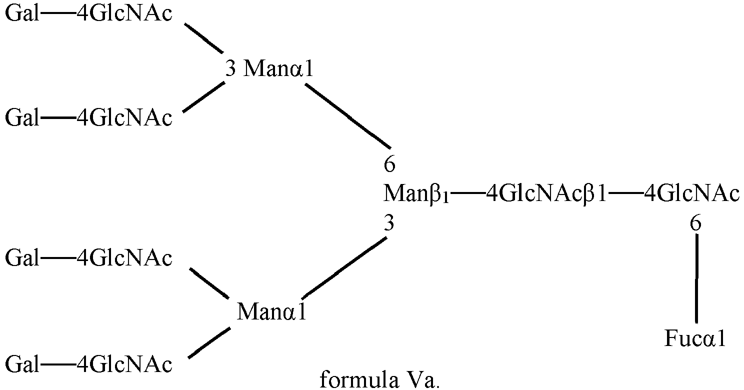

In a further embodiment, the Factor VII or Factor VIIa glycoprotein described immediately above has a glycosylation pattern that further comprises:
(1) a glycan of formula IVa

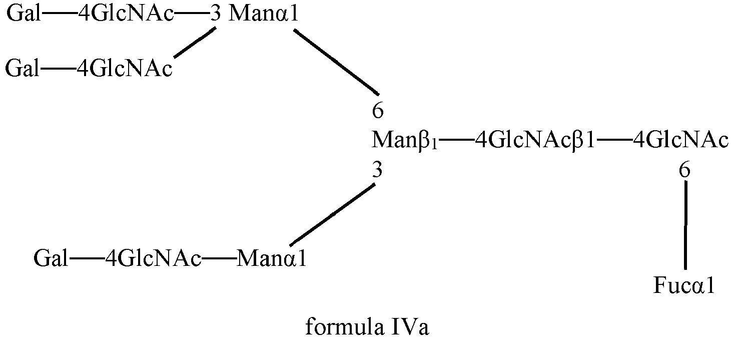

The Factor VII or Factor VIIa glycoprotein described above may have trace amounts or less of the glycan of formula IIa and/or the glycan of formula IIIa. In another embodiment of the invention, the Factor VIIa glycoprotein comprises a heavy chain; wherein the heavy chain has an N-linked glycosylation pattern comprising glycans; and further wherein the glycosylation pattern comprises:
(1) substantially no glycans with terminal N-acetylneuraminic acid and
(2) a substantial glycan that is a glycan of formula la: 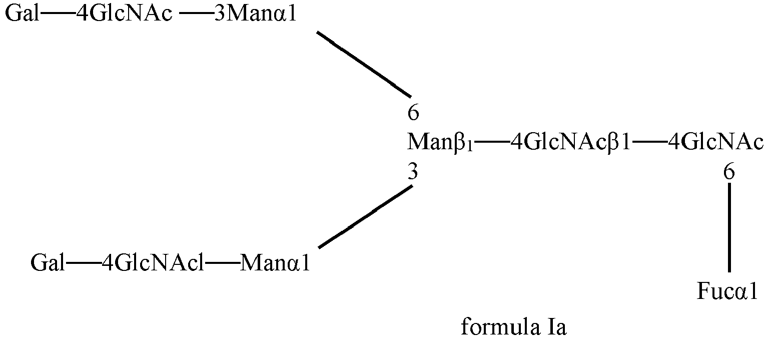 and
(3) trace amounts or less of one or more glycans selected from the group consisting of a glycan of formula IIa: 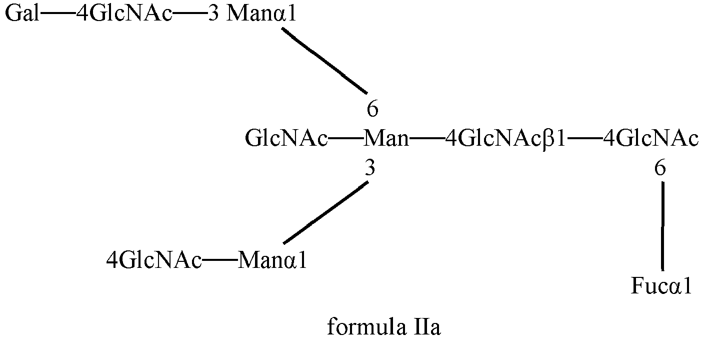 and a glycan of formula IIIa:

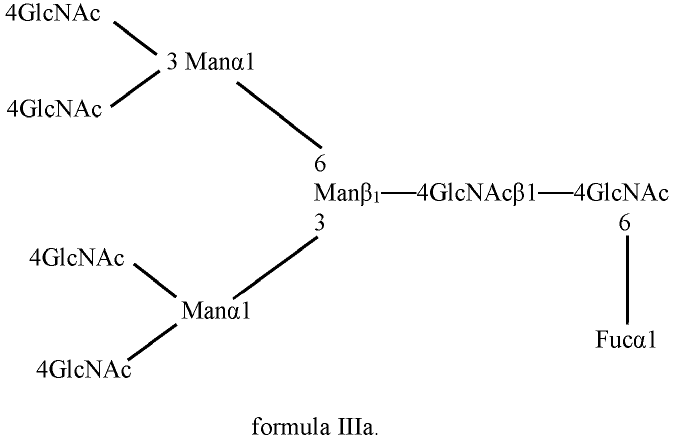

In certain embodiments, the amino acid sequence of the Factor VII or Factor VIIa glycoprotein comprises, contains, consists essentially of or consists of the amino acid sequence of wild type human Factor VII or Factor VIIa (SEQ ID NO: 2). In one embodiment, the glycoprotein has an amino acid sequence with 1, 2, 3, 4, 5, 6, 7, 8, 9 or 10 amino acid differences from the amino acid sequence of SEQ ID NO: 2, and preferably has conservative amino acid substitutions when substitutions are present. In another embodiment, the amino acid sequence of the Factor VII or Factor VIIa glycoprotein comprises, contains, consists essentially of or consists of the amino acid sequence of SEQ ID NO: 2 and has an amino acid sequence with 1, 2, 3, 4, 5, 6, 7, 8, 9 or 10 amino acid differences from the amino acid sequence of SEQ ID NO: 2, provided that the amino acid sequence does not comprise or consist of the amino acid sequence of SEQ ID NO: 2 having the mutations P10Q, K32E, A34E, R36E, T106N, and V253. In one embodiment, the glycoprotein has an amino acid sequence with 1, 2, 3, 4, 5, 6, 7, 8, 9 or 10 amino acid differences from the amino acid sequence of SEQ ID NO: 2 and the amino acid sequence does not comprise or consist of the amino acid sequence of SEQ ID NO: 2 having only the mutations P10Q, K32E, T106N and V253N, or only the mutations P10Q, K32E, A34E, R36E, T106N, and V253N. It is to be understood that "having only the mutations" means that amino acid sequences with additional mutations as well as the listed mutations are outside the scope of the disclaimer.

In another embodiment, the glycoprotein has an amino acid sequence that has 99, 98, 97, 96, 95, 94, 93, 92, 91, 90, 89, 88, 87, 86, 85, 84, 83, 82, 81, 80, 75, or 70% homology with the amino acid sequence of SEQ ID NO: 2 and has Factor VII or Factor VIIa activity. In another embodiment, the glycoprotein has an amino acid sequence that has 99, 98, 97, 96, 95, 94, 93, 92, 91, 90, 89, 88, 87, 86, 85, 84, 83, 82, 81, 80, 75, or 70% homology with the amino acid sequence of SEQ ID NO: 2 and has factor VII or Factor VIIa activity, provided that the amino acid sequence does not comprise or consist of SEQ ID NO: 2 having only mutations P10Q, K32E, A34E, R36E, T106N, and V253. In a further embodiment, the glycoprotein has an amino acid sequence that has 99, 98, 97, 96, 95, 94, 93, 92, 91, 90, 89, 88, 87, 86, 85, 84, 83, 82, 81, 80, 75, or 70% homology with the amino acid sequence of SEQ ID NO: 2 and the amino acid sequence does not comprise or consist of the amino acid sequence of SEQ ID NO: 2 having mutations at only P10Q, K32E, T106N and V253N, or at only P10Q, K32E, A34E, R36E, T106N, and V253N.

In another embodiment the Factor VII or Factor VIIa glycoprotein is encoded by a nucleic acid sequence that encodes the amino acid sequence of SEQ ID NO: 2, or encodes a polypeptide having an amino acid sequence having 99, 98, 97, 96, 95, 94, 93, 92, 91, 90, 89, 88, 87, 86, 85, 84, 83, 82, 81, 80, 75, or 70% homology with the amino acid sequence of SEQ ID NO: 2 and having Factor VII or Factor VIIa activity. In another embodiment, the Factor VII or Factor VIIa glycoprotein is encoded by a nucleic acid sequence that encodes the amino acid sequence of SEQ ID NO: 2, or encodes a polypeptide having an amino acid sequence having 99, 98, 97, 96, 95, 94, 93, 92, 91, 90, 89, 88, 87, 86, 85, 84, 83, 82, 81, 80, 75, or 70% homology with the amino acid sequence of SEQ ID NO: 2 and having Factor VII or Factor VIIa activity, and the amino acid sequence does not comprise or consist of the amino acid sequence of SEQ ID NO: 2 having the mutations P10Q, K32E, T106N and V253N, or having the mutations P10Q, K32E, T106N and V253N and P10Q, K32E, A34E, R36E, T106N, and V253N.

In one embodiment the Factor VII or Factor VIIa glycoprotein is encoded by a nucleic acid that comprises, contains, consists essentially of, or consists of the nucleotide sequence encoding wild type human Factor VII (SEQ ID NO: 1). In one embodiment, the nucleotide sequence encoding the glycoprotein has 1, 2, 3, 4, 5, 6, 7, 8, 9 or 10 nucleic acid differences from the nucleotide sequence of SEQ ID NO: 1 and encodes a polypeptide that has Factor VII or Factor VIIa activity, in one embodiment provided that the amino acid sequence of the polypeptide does not consist of SEQ ID NO: 2 having only mutations P10Q, K32E, A34E, R36E, T106N, and V253; and in another embodiment provided that the amino acid sequence of the polypeptide does not have only the mutations P10Q, K32E, T106N and V253N. In another embodiment, the nucleotide sequence encoding the glycoprotein has a nucleotide sequence that has 99, 98, 97, 96, 95, 94, 93, 92, 91, 90, 89, 88, 87, 86, 85, 84, 83, 82, 81, 80, 75, or 70% homology with the nucleotide sequence of SEQ ID NO: 1 and encodes a protein having Factor VII or Factor VIIa activity, in one embodiment provided that the amino acid sequence of the protein does not consist of SEQ ID NO: 2 having only mutations P10Q, K32E, A34E, R36E, T106N, and V253N; and in another embodiment provided that the amino acid sequence does not only having mutations P10Q, K32E, T106N and V253N. In another embodiment, the Factor VII glycoprotein is encoded by a nucleic acid that encodes wild type human Factor VII and whose sequence is determined by codon optimization for the host cell. In one embodiment, the nucleic acid encoding the Factor VII glycoprotein of the present disclosure comprises, contains, or consists of the nucleic acid sequence of SEQ ID NO: 3 or a nucleic acid sequence that comprises, contains, or consists of a nucleotide sequence that has 99, 98, 97, 96, 95, 94, 93, 92, 91, 90, 89, 88, 87, 86, 85, 84, 83, 82, 81, 80, 75, or 70% homology with the nucleotide sequence of SEQ ID NO: 3 and encodes a protein having Factor VII or Factor VIIa activity, in one embodiment provided that the amino acid sequence of the protein does not consist of SEQ ID NO: 2 having only mutations P10Q, K32E, A34E, R36E, T106N, and V253N; and in another embodiment provided that the amino acid sequence of the protein does not consist of SEQ ID NO: 2 having only mutations P10Q, K32E, T106N and V253N.

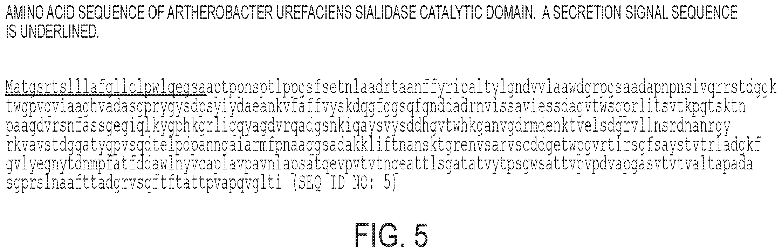

The glycoproteins of the present disclosure may be recombinantly produced in a Chinese Hamster Ovary (CHO) cell line that is a CHO K-1 or CHO-S cell line under conditions such as those in the examples or under other culture conditions that allow for suitable cell productivity for recombinant protein expression and recovery. Other suitable CHO cell lines may be identified by one of ordinary skill in the art by routine testing in view of the knowledge of various CHO cell lines. See, e.g., Wurm, Florian, "CHO Quasispecies - Implications for Manufacturing Processes," Processes (2013), 1, 296-311; doi: 10.3390/pr1030296. The CHO host cell is recombinantly transfected with a nucleic acid encoding Factor VII and a nucleic acid encoding sialidase. In certain embodiments, the sialidase nucleic acid is from the species *Artherobacter urefaciens* or derived therefrom, such as by codon optimization, for example, having the nucleic acid sequence of SEQ ID NO: 4, in either case expressing a sialidase comprising the amino acid sequence of SEQ ID NO: 5 as shown in Figure 5 with or without the secretion signal sequence. In another embodiment, the host cell is transfected with a nucleic acid encoding sialidase having at least 99%, 98%, 97%, 96%, 95%, 94%, 93%, 92%, 91%, 90%, 89%, 88%, 87%, 86%, 85%, or 80% identity to the amino acid sequence of SEQ ID NO: 5 with or without the secretion signal sequence. The transfected host cell may be cultured under the following conditions: CD-Fusion medium (Sigma-Aldrich) with 6 mM GlutaMAX ® media (Gibco), which contains L-glutamine, and 30 ng/mL vitamin K. It is believed the glycoproteins of the present disclosure may be produced from CHO host cells cultured in ActiCHO cell culture media (GE Healthcare) supplemented with glutamine and vitamin K as appropriate for the CHO cell line. Other media in which CHO cells can grow may potentially also be suitable (e.g., CD-CHO medium from ThermoFisher Scientific).

Pharmaceutical formulations of the Factor VII or Factor VIIa glycoproteins and compositions thereof comprising the Factor VII or Factor VIIa polypeptide and a pharmaceutically acceptable excipient or carrier are also useful. In certain examples, the pharmaceutical formulations are for parenteral administration, such as by intravenous, subcutaneous or intramuscular administration, and dosing may be as a single bolus dose, intermittent dosing, or as a continuous intravenous infusion. Topical formulations are also useful. One embodiment comprises a pharmaceutical formulation comprising a Factor VII or Factor VIIa glycoprotein as described herein in a lyophilized preparation that is reconstituted at the time of use. Alternatively, the pharmaceutical formulation can be a stable liquid ready-to-use formulation not requiring reconstitution. The pharmaceutical formulation can be a lyophilized powder in single-use vials of 1, 2, 5, or 8 mg of Factor VII polypeptide. After reconstitution with a specified volume of liquid, such as sterile water containing histidine, the final solution can contain any suitable amount of Factor VII or Factor VIIa glycoprotein that produces a therapeutic effect, such as, without limitation, 0.2 mg/mL, 0.5 mg/mL, 1 mg/mL (1000 micrograms/mL), 2 mg/mL, 3 mg/mL, 4 mg/mL, 5 mg/mL, 1-2 mg/mL, 1-3 mg/mL, 1-5 mg/mL, 1-10 mg/mL, 0.2-1.0 mg/mL, 0.5-1 mg/mL, 0.5-2 mg/mL, 0.5-5 mg/mL, 0.5-4 mg/mL, or 0.5-10 mg/mL of Factor VIIa or Factor VII glycoprotein. Proper dosage for administration to a patient can be readily determined by persons skilled in the art based upon, for example, the weight of the patient, the type of bleeding disorder or episode being treated, and the activity of the particular Factor VIIa or Factor VII polypeptide being employed. In certain examples, dosing can be in the range of 70-110 micrograms/kg, 70-90 micrograms/kg, or 80-100 micrograms/kg and can be 90 micrograms/kg. The lyophilized powder may be reconstituted with an aqueous carrier, such as water, buffered water, 0.4% saline, 0.3% glycine, etc. Actual methods for preparing parenterally administrable compositions will be known or apparent to those skilled in the art and are described in more detail in, for example, Remington's Pharmaceutical Sciences, 18th ed., Mack Publishing Company, Easton, Pa. (1990). Topical application, such as can be advisable in the case of trauma, can be carried out by means of a spray, perfusion, catheters, stent, vascular graft or stent, ointment, or other preparation known in the art. In certain examples, topical administration can be by way of a sold or semi-solid matrix, such as a surgical sponge or collagen matrix, which has been treated with, infused with, coated with, or soaked in a composition comprising the Factor VIIa variant. Methods of preparing such matrices are well known in the art (see, e.g., Thrombosis/Hemostasis 12:445, 2006) and the skilled artisan would be able to readily determine an appropriate dose and method of application of the composition onto the given matrix.

The present disclosure relates to kits comprising the Factor VII or Factor VIIa glycoprotein. In certain examples, the kit contains a vial containing ready-to-use liquid containing the glycoprotein in a suitable pharmaceutical composition. In other examples, the kit contains a vial containing lyophilized Factor VII or Factor VIIa glycoprotein, or a lyophilized formulation comprising the glycoprotein, and also a diluent for reconstitution. In other examples, the kit contains a topical formulation of the Factor VII or Factor VIIa glycoprotein, for example, an ointment, spray, or liquid, and a matrix such as a sponge or other medical matrix to which the topical formulation may be applied before administration to the patient.

The Factor VII or Factor VIIa glycoproteins and compositions described herein are useful for use in the treatment of blood clotting disorders, and those disorders that benefit from blood coagulation, particularly for coagulation with a drug having a shorter half-life than wild type Factor VII, and are useful in making medicaments to treat these disorders. Accordingly, the Factor VII or Factor VIIa glycoproteins and compositions herein are useful for hemorrhage, penetrating traumatic injury; blunt traumatic injury; bleeding in elective surgery; bleeding in cardiac surgery; bleeding in spinal surgery; orthopedic surgery; neurosurgery; oncology surgery; postpartum surgery; postpartum hemorrhage; menorrhagia; bleeding in stem cell transplantation; bleeding in liver transplantation; gastrointestinal bleeding; active variceal bleeding in cirrhosis; non variceal bleeding in cirrhosis; diffuse alveolar hemorrhage; aortic aneurysm; intracerebral hemorrhage; traumatic brain injury; brain contusion; reversal of warfarin; reversal of heparin; reversal of anticoagulants; reversal of anti-thrombotics; Factor VII deficiency; burns; prophylaxis in hemophilia patients with inhibitors; partial hepatectomy for non-cirrhotic and cirrhotic patients; acquired hemophilia; idiopathic thrombocytopenic purpura; Glanzmann's Thrombasthenia; Glanzmann's Thrombasthenia refractory to platelet transfusion and Bernard-Soulier Syndrome. In certain embodiments, the Factor VII glycoforms of the present disclosure are useful for treating hemorrhage, gastrointestinal bleeding, uncontrolled bleeding, bleeding in a mammal undergoing transplantation or resection or surgery, variceal bleeding, thrombocytopenia, hemophilia, intracranial hemorrhage, aortic aneurysm, and over administration of an anticoagulant.

Postpartum hemorrhage is a leading cause of maternal mortality. Thus, there is a need for effective treatment that can be met by the Factor VII glycoforms of the present disclosure. Postpartum hemorrhage has been defined as a blood loss of more than 500 mL following vaginal delivery or more than 1000 mL following cesarean delivery. A woman with pre-existing anemia or certain other disorders may have less ability to cope with such a blood loss and so may suffer from postpartum hemorrhage with lower amounts of blood loss than those identified above. In view of this, some clinicians suggest that postpartum hemorrhage should be defined to include any amount of blood loss that threatens the hemodynamic stability of the woman. Rapid recognition and diagnosis of postpartum hemorrhage followed by rapid initiation of appropriate management is essential to successful management of the disorder.

### Examples

A Chinese Hamster Ovary (CHO-K1) cell line acquired from SAFC (Sigma-Aldrich) was used as the parent cell line for transfection. A cryopreserved cell bank of this CHO-K1 cell line was prepared in CD-Fusion medium and sterility, mycoplasma and non-endogenous virus (adventitious virus and minute mouse virus) were tested before its being used for cell line construction.

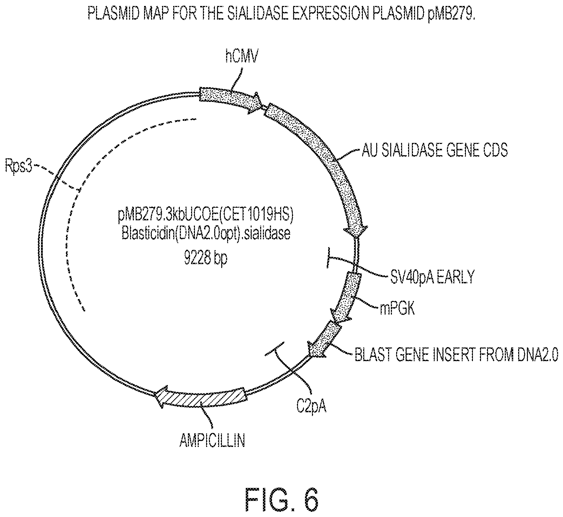

Cell line construction was performed in CD-Fusion medium with 6 mM GlutaMAX ® media (Gibco), and 30 ng/mL vitamin K. The CHO-K1 cells were transfected with linearized pMB246 plasmid DNA using an Amaxa Nucleofector II device with an Amaxa Cell Line Nucleofector Kit V (Lonza Biologics, Slough, UK) using the U-023 program. The pMB246 plasmid contained a nucleic acid sequence encoding human wild type Factor VII of SEQ ID NO: 2. The nucleic acid was codon optimized. Twenty-four hours after transfection the transfectants were directly plated out in 96-well plates with CD-Fusion selection medium containing 10 ug/ml puromycin (Invitrogen). Following drug selection and screening cells from the best-producing, drug-resistant, single foci wells were selected for expansion. After further characterization of the top clones based on specific chromogenic activity the final candidate cell line was selected and a Research Cell Bank (RCB) was produced. These cells were super-transfected with linearized pMB279 plasmid DNA (see Figure 6) using a similar method as described above. Twenty-four hours after transfection the cells were directly plated out in 96-well plates and with CD-Fusion selection medium containing 30 or 50 ug/ml blasticidin selection antibiotic (Invitrogen). After drug selection and screening, the drug-resistant, single foci wells were selected for expansion. After further characterization of the top eight clones based on protein quality and productivity, the final five candidate cell lines were selected and Research Cell Banks (RCB) of those five cell lines were generated.

Both sialidase and Factor VII are secreted from the engineered cell line. As discussed in detail below, the Factor VII zymogen in the harvest was purified and activated to active Factor VIIa using a modification of known methods. See, e.g., U.S. Pat. App. Pub. 20090047723, "Method for Purification of Factor VII," (Rikke Bolding Jensen and Frank Bech Nygaard).

Factor VII expressed in mammalian cells has a number of post-translational modifications, including modification of up to 10 glutamate residues within its N-terminal Gla domain by vitamin K-dependent carboxylation to form γ-carboxyglutamate (Gla) residues. Since Factor VII can be expressed with a variable number of Gla residues, a key goal of the purification is to isolate desialylated Factor VII with a high content of Gla modifications (mostly 9 to 10 for wild type).

In the first step of purification, the harvest media was changed into a buffer solution, concentrated and frozen for later processing. The second step, capture of the product by anion exchange chromatography (AIEC), allows for further concentration of the product and preparation for the third step, hydroxyapatite chromatography (HAC). This later step provides a substantial purification of the product, and can also remove forms of FVII present with a low content of Gla modifications. A fourth step is a second AIEC, which prepares the protein product for the in-solution activation step such as by altering protein concentration and changing the buffer.

Because Factor VII is expressed in the single chain zymogen form, an activation step is needed to activate the protein by cleavage between the R and the I (residue 153 of the mature protein). An "in solution activation step" was used that involves an incubation of the protein at room temperature under conditions that promote the auto proteolytic cleavage of Factor VII to Factor VIIa. The activation step was followed with a polishing step (third AIEC) and dialysis of the final product into formulation buffer. The final protein is stored at -70 deg C.

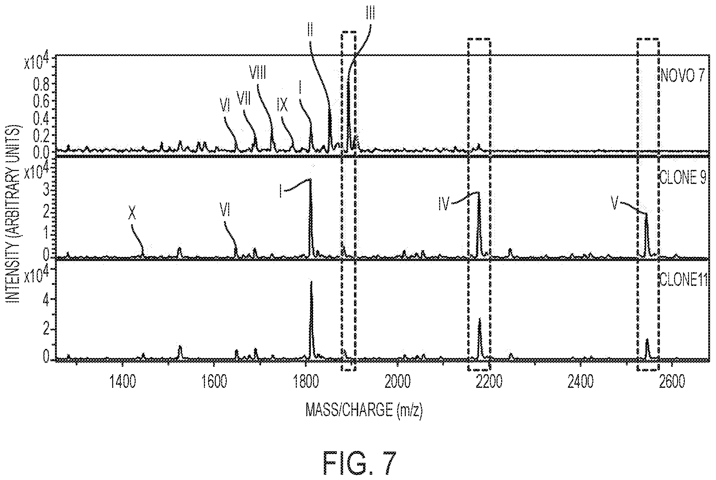

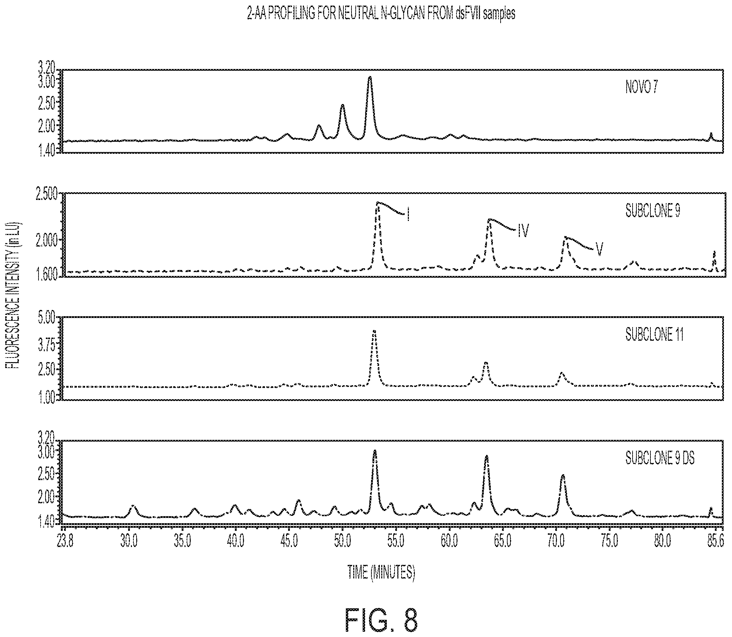

N-linked glycans were removed by enzyme treatment, and the glycans were analyzed using MALDI mass spectrometry. The results are shown in Figure 7. The legend for the Roman numerals used in Figure 7 is found in Figure 3. As shown in Figure 7, the N-linked glycans from desialylated clones are mostly fucosylated bi-antennary (see, e.g., formula I peak) and tri- and tetra-antennary structures (see, e.g., formula IV and V peaks). The mass and the intensity of the peak can be used to determine the glycan composition, and the relative abundance of detectable glycan structures. The data in Figures 7 and 8 is for CHO-K1 cell line subclones 9, 11, and (for Figure 8) 9 DS, which is subclone 9 purified to a greater extent than the sample marked subclone 9, compared to desialylated NOVOSEVEN. Subclone 9 and subclone 11 were purified and auto-activated. Subclone 9 DS was further purified using tangential flow filtration. Thus all the samples were activated (i.e., desialylated wild type FVIIa).

Figure 8 shows analysis of glycans from NOVOSEVEN and from clones of the engineered CHO K-1 cell by the method of HPLC after 2-AA labeling. N-linked glycans are enzymatically released, labeled with 2-AA using standard methods, and then analyzed by HPLC. "ds" as in the title of Figure 8 refers to a glycoprotein substantially desialylated. Glycans were quantified as shown in the following table:

| | **Percent of total glycans** | | | |
|---|---|---|---|---|
| **FVIIa clone** | **I** | **IV** | **V** | **other** |
| Novo7 | 45% | nd* | nd | 35 |
| Clone 9 | 34.1 | 35.7 | 24.2 | 6 |
| Clone 11 | 45.8 | 28.0 | 13.9 | 12.2 |
| Clone 9 DS | 27.1 | 26.9 | 18.0 | 28 |

| | | | | |
|---|---|---|---|---|
| nd= not detectable | | | | |

The N-glycosylation pattern of the heavy chain of recombinant Factor VIIa was analyzed using LC-mass spectrometry for structural characterization, resolved by HPLC after reduction of the activated Factor VIIa molecule. These data therefore, show only those N-linked glycans on the HC, which are a subset of the total pool of N-linked glycans present on the intact HC and HL heterodimer. Figure 9 reports the glycosylation profile for the desialylated heavy chain ("HC") of the reference product, NOVOSEVEN recombinant Factor VIIa and that of a Factor VIIa glycoprotein of the present disclosure. Desialylated NOVOSEVEN recombinant Factor VIIa may be produced by gentle mixing of a slurry of neuraminidase-agarose beads (Sigma N5254) and a buffer solution of rFVIIa at room temperature for about 16-24 hours. One of ordinary skill can envision additional methods to generate such an enzymatic desialylation. The N-linked glycosylation on the heavy chain of the reference desialylated NOVOSEVEN Factor VIIa product is mostly bi-antennary structure with bisecting and different sialylation (terminal sialic acid content: 0, 1, or 2). Very small amounts of tri-antennary structure were observed.

In contrast, the heavy chain of the Factor VIIa glycoprotein of the present disclosure has a different glycosylation pattern from the reference desialylated NOVOSEVEN product. Figure 9 (top panel) shows the N-glycan distribution for the HC on a desialylated Factor VIIa according to the present invention produced in a research cell line using a CHO-S cell transfected with nucleotides encoding recombinant wild type human Factor VII and sialidase active domain of *A urefaciens.* In Figure 9, the downward arrows show two glycans present in desialylated NOVOSEVEN but substantially or completely absent from the Factor VII glycoforms of the present invention. As shown in Figure 9, the glycoprotein of the present invention, when analyzed by LC-MS, shows no glycoforms corresponding to formula II and formula III. However, the HC of the reference product shows significant amounts of each of these glycoforms.

Subclone 9 DS retained activity in vitro as measured by phospholipid-Factor Xa (PL-Xa) activation. Activity of Factor VIIa may also be assessed using standard thrombin generation assay conditions in normal pooled human plasma. 1 pM tissue factor is the activator and the sample is run under 4 micromolar lipid concentration. Clearance may be measured in an in vitro hepatocyte clearance assay. Increased clearance may be measured in vivo in rat and in a HemA PK study.

The Factor VII and Factor VIIa glycoproteins of the present disclosure have glycans that can serve as ligands for clearance receptors, such as the asialoglycoprotein receptor (ASGPR) in the liver. This receptor binds to glycans having terminal galactose residues. In particular, ASGPR binds glycans with multiple arms terminating with man-GlcNAc-Gal. The presently disclosed glycoproteins have reduced levels of glycans found on other recombinant Factor VII and Factor VIIa molecules such as NOVOSEVEN recombinant Factor VIIa, or glycans ending in GlcNAc. These glycans have the potential for interaction with other cell receptors, such as Man/GlcNAc receptors that facilitate uptake into macrophages and other cells, such as dendritic cells. Without wanting to be limited by theory in any way, it is believed that such glycans can promote a different mechanism of clearance, compared to the presently disclosed glycoproteins, and therefore, have a different clinical utility. For example, the rate of uptake into macrophages can be different than uptake by the ASGPR, resulting in different duration of actions and bio-distributions. Importantly, uptake into dendritic cells via Man/GlcNAc receptors has been associated with higher rates of immunogenicity. The role of different glycan structures may be particularly important for clearance of molecules further altered to remove sialic acid, such as those of the present invention.

## Claims

1. A Factor VII or Factor FVIIa glycoprotein having an N-linked glycosylation pattern comprising glycans; wherein the glycosylation pattern comprises:
(1) substantially no glycans with terminal N-acetylneuraminic acid and
(2) a substantial glycan that is a glycan of formula I: (GlcNAc)₄(Man)₃(Gal)₂(Fuc) and
(3) trace amounts or less of one or more glycans selected from the group consisting of a glycan of formula II: (GlcNAc)₅(Man)₃(Gal)(Fuc) and a glycan of formula III: (GlcNAc)₆(Man)₃ (Fuc) and;
(4) a glycan of formula V: (GlcNAc)₆(Gal)₄(Man)₃(Fuc).

2. The Factor VII or Factor VIIa glycoprotein of claim 1, wherein the glycosylation pattern further comprises a glycan of formula IV: (GlcNAc)₅(Man)₃(Gal)₃(Fuc).

3. The Factor VII or Factor VIIa glycoprotein of claim 1 or 2, wherein the glycosylation pattern has trace amounts or less of the glycan of formula II.

4. The Factor VII or Factor VIIa glycoprotein of claim 1 or 2, wherein the glycosylation pattern has trace amounts or less of the glycan of formula III.

5. The Factor VII or Factor VIIa glycoprotein of any one of claims 1-4, wherein the glycoprotein comprises an amino acid sequence having three or less mutations relative to SEQ ID NO: 2.

6. The Factor VII or Factor VIIa glycoprotein of claim 1, wherein the glycoprotein is a Factor VIIa glycoprotein and comprises a heavy chain; wherein the heavy chain has an N-linked glycosylation pattern comprising glycans; and further wherein the glycosylation pattern comprises:
(1) substantially no glycans with terminal N-acetylneuraminic acid and
(2) a substantial glycan that is a glycan of formula I: (GlcNAc)₄(Man)₃(Gal)₂(Fuc); and
(3) trace amounts or less of one or more glycans selected from the group consisting of a glycan of formula II: (GlcNAc)₅(Man)₃(Gal)(Fuc)and a glycan of formula III: (GlcNAc)₆(Man)₃(Fuc).

7. The Factor VIIa glycoprotein of claim 6, wherein the glycosylation pattern has trace amounts or less of the glycan of formula II.

8. The Factor VIIa glycoprotein of claim 5 or 6, wherein the glycosylation pattern has trace amounts or less of the glycan of formula III.

9. The Factor VIIa glycoprotein of any one of claims 5-8, wherein the glycoprotein comprises an amino acid sequence having three or less mutations relative to SEQ ID NO: 2.

10. A method of making the Factor VII or Factor VIIa glycoprotein of one of claims 2-9 comprising:
expressing a recombinant nucleic acid encoding the amino acid sequence for the Factor VII or Factor VIIa glycoprotein in a CHO K-1 host cell that has been recombinantly engineered to express sialidase; and
isolating the Factor VII or Factor VIIa glycoprotein thereby produced.

11. The method of claim 10, wherein the sialidase comprises an amino acid sequence of SEQ ID NO: 5.

12. The Factor VII glycoprotein of any one of claims 1-9 for use in a method for treating a mammal having a disease or a disorder wherein blood clot formation is desirable, wherein the disease or disorder is selected from the group consisting of a hemorrhage, gastrointestinal bleeding, uncontrolled bleeding, bleeding in a mammal undergoing transplantation or resection or surgery, variceal bleeding, thrombocytopenia, hemophilia, intracranial hemorrhage, aortic aneurysm, and over administration of an anticoagulant, penetrative traumatic injury, blunt traumatic injury, bleeding in elective surgery, bleeding in cardiac surgery, bleeding in spinal surgery, orthopedic surgery, neurosurgery, oncology surgery, post-partum surgery, postpartum hemorrhage, menorrhagia, bleeding in stem cell transplantation, bleeding in liver transplantation, gastrointestinal bleeding, active variceal bleeding in cirrhosis, non variceal bleeding in cirrhosis, diffuse alveolar hemorrhage, aortic aneurysm, intracerebral hemorrhage, traumatic brain injury, brain contusion, reversal of warfarin, reversal of heparin, reversal of anticoagulants, reversal of anti-thrombotics, Factor VII deficiency, burns, prophylaxis in hemophilia patients with inhibitors, partial hepatectomy for non-cirrhotic and cirrhotic patients, acquired hemophilia, idiopathic thrombocytopenic purpura, Glanzmann's Thrombasthenia, Glanzmann's Thrombasthenia refractory to platelet transfusion, and Bernard-Soulier Syndrome.

13. The Factor VII glycoprotein for use according to claim 12, wherein the disease or disorder is hemorrhage that is postpartum hemorrhage.

14. A pharmaceutical composition comprising the Factor VII or Factor VIIa glycoprotein of any one of claims 1-9 and a pharmaceutically acceptable excipient.

## Patentansprüche

1. Ein Faktor VII- oder Faktor FVIIa-Glykoprotein mit einem N-verknüpften Glykosylierungsmuster, das Glykane enthält; **dadurch gekennzeichnet, dass** das Glykosylierungsmuster folgende Bestandteile enthält:
(1) im Wesentlichen keine Glykane mit terminaler N-Acetylneuraminsäure und
(2) ein wesentliches Glykan mit Struktur nach Formel I: (GlcNAc)₄(Man)₃(Gal)₂(Fuc) und
(3) Spurenmengen oder weniger von einem oder mehreren Glykanen enthält, die aus der Gruppe von Glykanen mit Struktur nach Formel II ausgewählt wurden: (GlcNAc)₅(Man)₃(Gal)(Fuc) und ein Glykan mit Struktur nach Formel III: (GlcNAc)₆(Man)₃(Fuc) und
(4) ein Glykan mit Struktur nach Formel V: (GlcNAc)₆(Gal)₄(Man)₃(Fuc).

2. Das Faktor VII- oder Faktor VIIa- Glykoprotein des Anspruchs 1, **dadurch gekennzeichnet, dass** das Glykosylierungsmuster noch folgende Elemente enthält: ein Glykan mit Struktur nach Formel IV: (GlcNAc)₅(Man)₃(Gal)₃(Fuc).

3. Das Faktor VII- oder Faktor VIIa-Glykoprotein nach Anspruch 1 oder 2, **dadurch gekennzeichnet, dass** das Glykosylierungsmuster Spurenmengen oder weniger Glykan mit Struktur nach Formel II enthält.

4. Das Faktor VII- oder Faktor VIIa-Glykoprotein nach Anspruch 1 oder 2, **dadurch gekennzeichnet, dass** das Glykosylierungsmuster Spurenmengen oder weniger von Glykan mit Struktur nach Formel III enthält.

5. Das Faktor VII- oder Faktor VIIa-Glykoprotein nach einem der Ansprüche 1-4, **dadurch gekennzeichnet, dass** das Glykoprotein eine Aminosäuresequenz enthält, in der drei oder weniger Mutationen im Verhältnis zu SEQ ID Nr. 2 vorhanden sind.

6. Das Faktor VII- oder Faktor VIIa-Glykoprotein nach Anspruch 1, **dadurch gekennzeichnet, dass** das Glykoprotein ein Faktor VIIa-Glykoprotein ist und aus einer schweren Kette besteht; **dadurch gekennzeichnet, dass** die schwere Kette ein N-verknüpftes Glykosylierungsmuster aus Glykanen enthält; und weiter **dadurch gekennzeichnet, dass** das Glykosylierungsmuster aus folgenden Elementen besteht:
(1) im Wesentlichen keine Glykane mit terminaler N-Acetylneuraminsäure und
(2) ein wesentliches Glykan mit Struktur nach Formel I: (GlcNAc)₄(Man)₃(Gal)₂(Fuc);
und
(3) Spurenmengen oder weniger von einem oder mehreren Glykanen enthält, die aus der Gruppe von Glykanen mit Struktur nach Formel II ausgewählt wurden: (GlcNAc)₅(Man)₃(Gal)(Fuc) und ein Glykan mit Struktur nach Formel III: (GlcNAc)₆(Man)₃(Fuc).

7. Das Faktor VIIa-Glykoprotein nach Anspruch 6, **dadurch gekennzeichnet, dass** das Glykosylierungsmuster Spurenmengen oder weniger Glykan mit Struktur nach Formel II enthält.

8. Das Faktor VIIa-Glykoprotein nach Anspruch 5 oder Anspruch 6, **dadurch gekennzeichnet, dass** das Glykosylierungsmuster Spurenmengen oder weniger Glykan mit Struktur nach Formel III enthält.

9. Das Faktor VIIa-Glykoprotein nach einem der Ansprüche 5-8, **dadurch gekennzeichnet, dass** das Glykoprotein eine Aminosäuresequenz enthält, in der drei oder weniger Mutationen im Verhältnis zu SEQ ID Nr. 2 vorhanden sind.

10. Eine Methode zur Herstellung des Faktor VII- oder Faktor VIIa-Glykoproteins nach einem der Ansprüche 2-9, die folgende Verfahren beinhaltet:
Expression einer rekombinanten Nukleinsäure, die für die Aminosäuresequenz für das Faktor VII oder Faktor VIIa Glykoprotein in einer CHO K-1 Wirtszelle kodiert, die für die Expression von Sialidase rekombinant hergestellt wurde; und
Isolierung des Faktor VII- oder Faktor VIIa- Glykoproteins, das dabei entstanden ist.

11. Die Methode nach Anspruch 10, **dadurch gekennzeichnet, dass** die Sialidase eine Aminosäuresequenz nach SEQ ID Nr. 5 enthält.

12. Das Faktor VII-Glykoprotein nach einem der Ansprüche 1-9 zur Benutzung in einer Methode zur Behandlung eines Säugetiers mit einer Krankheit oder einer medizinischen Störung, **dadurch gekennzeichnet, dass** eine Blutgerinnselbildung erwünscht ist, **dadurch gekennzeichnet, dass** die Krankheit oder die medizinische Störung aus der Gruppe ausgewählt ist, die aus folgenden Elementen besteht: Blutung, Magen-Darm-Blutung, unkontrollierte Blutung, Blutungen bei einer Säugetier-Transplantation oder Resektion oder Operation, Varizenblutung, Thrombozytopenie, Hämophilie, intrakranielle Blutung, Aortenaneurysma, Überdosierung eines Antikoagulans, penetrative traumatische Verletzung, stumpfe traumatische Verletzung, Blutung bei einer elektiven OP, Blutung bei einer Herz-OP, Blutung bei einer Wirbelsäulen-OP, orthopädische OP, Neurochirurgie, onkologische Chirurgie, postpartale Chirurgie, Menorrhagie, Blutung bei einer Stammzelltransplantation, Blutung bei einer Lebertransplantation, gastrointestinale Blutung, aktive Varizenblutung bei Leberzirrhose, nicht-varizeale Blutung bei Leberzirrhose, diffuse alveolarw Blutung, Aortenaneurysma, intrazerebrale Blutung, Schädel-Hirn-Trauma, Gehirnerschütterung, Umkehrung von Warfarin, Umkehrung von Heparin, Umkehrung von Antikoagulanzien, Umkehrung von Antithrombotika, Faktor VII-Mangel, Verbrennungen, Prophylaxe bei Hämophilie-Patienten mit Hemmstoffen, partielle Leberresektion für Patienten mit und ohne Leberzirrhose, erworbene Hämophilie, idiopathische thrombozytopenische Purpura, Thrombasthenie Glanzmann, Thrombasthenie Glanzmann refraktär zu Thrombozytentransfusionen und Bernard-Soulier-Syndrom.

13. Das Faktor VII-Glykoprotein zur Anwendung nach Anspruch 12, **dadurch gekennzeichnet, dass** die Krankheit oder die medizinische Störung eine postpartale Blutung ist.

14. Eine pharmazeutisch Zusammensetzung, die das Faktor VII- oder Faktor VIIa-Glykoprotein nach einem der Ansprüche 1-9 und einen pharmazeutisch annehmbaren Hilfsstoff enthält.

## Revendications

1. Une glycoprotéine du facteur VII ou du facteur FVIIa comprenant un motif de glycosylation N-lié comprenant des glycanes ; dans laquelle le motif de glycosylation comprend :
(1) substantiellement aucun glycane avec acide N-acétylneuraminique terminal et
(2) un glycane substantiel qui est un glycane de formule I : (GlcNAc)₄(Man)₃(Gal)₂(Fuc) et
(3) des quantités à l'état de traces ou moins d'un ou plusieurs glycanes choisis dans le groupe constitué par un glycane de formule II : (GlcNAc)₅(Man)₃(Gal)(Fuc) et un glycanes de formule III : (GlcNAc)₆(Man)₃(Fuc) et
(4) un glycane de formule V: (GlcNAc)₆(Gal)₄(Man)₃(Fuc).

2. La glycoprotéine du facteur VII ou du facteur VIIa selon la revendication 1, dans laquelle le motif de glycosylation comprend en outre : un glycane de formule IV : (GlcNAc)₅(Man)₃(Gal)₃(Fuc).

3. La glycoprotéine du facteur VII ou du facteur VIIa selon la revendication 1 ou 2, dans laquelle le schéma de glycosylation comprend des quantités à l'état de traces ou moins le glycane de formule II.

4. La glycoprotéine du Facteur VII ou du Facteur VIIa selon la revendication 1 ou 2, dans laquelle le schéma de glycosylation a des quantités à l'état de traces ou moins leu glycane de formule III.

5. La glycoprotéine du facteur VII ou du facteur VIIa selon l'une quelconque des revendications 1 à 4, dans laquelle la glycoprotéine comprend une séquence d'acides aminés présentant trois mutations ou moins par rapport à la SEQ ID NO : 2.

6. La glycoprotéine du Facteur VII ou du Facteur VIIa selon la revendication 1, dans laquelle la glycoprotéine est une glycoprotéine du Facteur VIIa et comprend une chaîne lourde ; dans laquelle la chaîne lourde comprend un motif de glycosylation N-liée comprenant des glycanes ; et en outre dans laquelle le motif de glycosylation comprend :
(1) substantiellement aucun glycane avec acide N-acétylneuraminique et
(2) un glycane substantiel qui est un glycane de formule I : (GlcNAc)₄(Man)₃(Gal)₂(Fuc) ;
et
(3) des quantités à l'état de traces ou moins d'un ou plusieurs glycanes choisis dans le groupe constitué par un glycanes de formule II : (GlcNAc)₅(Man)₃(Gal)(Fuc)et un glycanes de formule III : (GlcNAc)₆(Man)₃(Fuc).

7. La glycoprotéine de facteur VIIa selon la revendication 6, dans laquelle le schéma de glycosylation contient des quantités à l'état de traces ou moins du glycane de formule II.

8. La glycoprotéine du facteur VIIa selon la revendication 5 ou 6, dans laquelle le schéma de glycosylation contient des quantités à l'état de traces ou moins du glycane de formule III.

9. La glycoprotéine du facteur VIIa de l'une quelconque des revendications 5 à 8, dans laquelle la glycoprotéine comprend une séquence d'acides aminés ayant trois mutations ou moins par rapport à la SEQ ID NO : 2.

10. Un procédé de fabrication de la glycoprotéine du facteur VII ou du facteur VIIa selon l'une des revendications 2 à 9, comprenant les étapes suivantes :
expression d'un acide nucléique recombinant codant pour la séquence d'acides aminés de la glycoprotéine du facteur VII ou du facteur VIIa dans une cellule hôte CHO K-1 qui a été modifiée par recombinaison pour exprimer la sialidase ; et
isolation de la glycoprotéine du facteur VII ou du facteur VIIa ainsi produite.

11. Le procédé selon la revendication 10, dans lequel la sialidase comprend une séquence d'acides aminés de SEQ ID NO : 5.

12. La glycoprotéine du facteur VII de l'une quelconque des revendications 1 à 9 pour une utilisation dans une méthode de traitement d'un mammifère atteint d'une maladie ou d'un trouble dans la ou lequel la formation de caillots sanguins est souhaitable, dans laquelle la maladie ou le trouble est choisi(e) dans le groupe constitué par une hémorragie, une hémorragie gastro-intestinale, une hémorragie non contrôlée, une hémorragie chez un mammifère subissant une transplantation ou une résection ou une chirurgie, une hémorragie variqueuse, une thrombocytopénie, une hémophilie, une hémorragie intracrânienne, un anévrisme aortique et une administration excessive d'un anticoagulant, une blessure traumatique avec pénétration, une blessure traumatique contondante, une hémorragie lors d'une chirurgie élective, une hémorragie lors d'une chirurgie cardiaque, une hémorragie lors d'une chirurgie de la colonne vertébrale, une chirurgie orthopédique, une neurochirurgie, une chirurgie oncologique, une chirurgie post-partum, une ménorragie, une hémorragie lors d'une transplantation de cellules souches, hémorragie lors d'une transplantation hépatique, une hémorragie gastro-intestinale, une hémorragie variqueuse active lors d'une cirrhose, une hémorragie non variqueuse lors d'une cirrhose, une hémorragie alvéolaire diffuse, un anévrisme aortique, une hémorragie intracérébrale, une lésion cérébrale traumatique, une contusion cérébrale, une inversion de warfarine, une inversion d'héparine, une inversion d'anticoagulants, une inversion d'anti-thrombotiques, un déficit en facteur VII, des brûlures, une prophylaxie chez les patients hémophiles avec inhibiteurs, une hépatectomie partielle pour les patients non cirrhotiques et cirrhotiques, une hémophilie acquise, un purpura thrombocytopénique idiopathique, une thrombasthénie de Glanzmann, une thrombasthénie de Glanzmann réfractaire à la transfusion de plaquettes et un syndrome de Bernard-Soulier.

13. La glycoprotéine du facteur VII à utiliser selon la revendication 12, dans laquelle la maladie ou le trouble est une hémorragie qui est une hémorragie post-partum.

14. Une composition pharmaceutique comprenant la glycoprotéine du facteur VII ou du facteur VIIa selon l'une quelconque des revendications 1 à 9 et un excipient pharmaceutiquement acceptable.


### SEQUENCE LISTING

<110> Bayer HealthCare LLC Feldman, Richard I
<120> FACTOR VIIa GLYCOFORMS
<130> 0081563-000234
<160> 5
<170> PatentIn version 3.5
<210> 1
   <211> 1218
   <212> DNA
   <213> Homo sapiens
<400> 1
   gccaacgcgt tcctggagga gctgcggccg ggctccctgg agagggagtg caaggaggag        60
   cagtgctcct tcgaggaggc ccgggagatc ttcaaggacg cggagaggac gaagctgttc       120
   tggatttctt acagtgatgg ggaccagtgt gcctcaagtc catgccagaa tgggggctcc       180
   tgcaaggacc agctccagtc ctatatctgc ttctgcctcc ctgccttcga gggccggaac       240
   tgtgagacgc acaaggatga ccagctgatc tgtgtgaacg agaacggcgg ctgtgagcag       300
   tactgcagtg accacacggg caccaagcgc tcctgtcggt gccacgaggg gtactctctg       360
   ctggcagacg gggtgtcctg cacacccaca gttgaatatc catgtggaaa aatacctatt       420
   ctagaaaaaa gaaatgccag caaaccccaa ggccgaattg tggggggcaa ggtgtgcccc        480
   aaaggggagt gtccatggca ggtcctgttg ttggtgaatg gagctcagtt gtgtgggggg       540
   accctgatca acaccatctg ggtggtctcc gcggcccact gtttcgacaa aatcaagaac       600
   tggaggaacc tgatcgcggt gctgggcgag cacgacctca gcgagcacga cggggatgag       660
   cagagccggc gggtggcgca ggtcatcatc cccagcacgt acgtcccggg caccaccaac       720
   cacgacatcg cgctgctccg cctgcaccag cccgtggtcc tcactgacca tgtggtgccc       780
   ctctgcctgc ccgaacggac gttctctgag aggacgctgg ccttcgtgcg cttctcattg       840
   gtcagcggct ggggccagct gctggaccgt ggcgccacgg ccctggagct catggtcctc       900
   aacgtgcccc ggctgatgac ccaggactgc ctgcagcagt cacggaaggt gggagactcc       960
   ccaaatatca cggagtacat gttctgtgcc ggctactcgg atggcagcaa ggactcctgc      1020
   aagggggaca gtggaggccc acatgccacc cactaccggg gcacgtggta cctgacgggc      1080
   atcgtcagct ggggccaggg ctgcgcaacc gtgggccact ttggggtgta caccagggtc      1140
   tcccagtaca tcgagtggct gcaaaagctc atgcgctcag agccacgccc aggagtcctc      1200
   ctgcgagccc catttccc                                                    1218
<210> 2
   <211> 406
   <212> PRT
   <213> Homo sapiens
<400> 2
<210> 3
   <211> 1332
   <212> DNA
   <213> Homo sapiens
<400> 3
   atggtcagcc aagcccttcg cctcctctgt ctcctcctcg gccttcaagg atgtctcgca        60
   gcagtgttcg tgactcaaga agaggcccac ggagtgctgc acaggcgccg gagagcgaac       120
   gcgttcctgg aggagttgag gcccggctca ctggaacggg aatgcaagga ggagcagtgc       180
   tcatttgaag aggccagaga gattttcaag gatgcagaac gcaccaagtt gttctggatc       240
   tcctactcag acggagatca gtgcgcctcg tcgccgtgcc aaaacggagg ctcatgcaaa       300
   gaccagctcc agtcctacat ctgcttctgc ctgccggcgt tcgaagggcg gaactgtgaa       360
   actcacaagg atgaccagct catctgcgtt aacgaaaacg ggggatgcga acagtactgt       420
   agcgaccata ccggaaccaa gcgctcgtgc agatgccacg aaggttactc cctgcttgcc       480
   gacggcgtgt cgtgtactcc aaccgtcgag tacccgtgcg gaaagatccc tatcctggag       540
   aaacgcaacg catccaagcc tcagggacgg atcgtggggg ggaaggtctg tccaaaagga       600
   gaatgcccct ggcaagtgct gctcctcgtg aacggagctc agctgtgcgg gggaacgctc       660
   atcaatacca tctgggtggt gtcggccgcg cactgcttcg ataagatcaa gaactggcgg       720
   aatctgatcg ccgtgctggg agagcatgac ttgtccgagc acgacggcga tgaacagtcc       780
   agacgcgtcg cgcaagtgat catcccgtcg acttacgtgc cggggaccac caatcacgac       840
   atcgcccttt tgcgactcca tcagcctgtt gtgctgactg accacgtcgt cccgctctgt       900
   ctgccagaac ggacgttctc cgaaagaacc ctggcctttg tgaggtttag cctggtgagc       960
   ggatggggac aactgctgga caggggcgcg actgctctgg aactgatggt gcttaatgtg      1020
   ccacggctga tgacgcaaga ctgcctgcag cagtctcgta aggtcggcga ctcgccgaat      1080
   atcactgagt acatgttctg cgctggctac agcgatggca gcaaggattc atgtaaagga      1140
   gatagcggtg gcccacatgc aactcactat cgcggtacct ggtacctgac cggaattgtc      1200
   tcgtggggac aaggttgcgc taccgtcggt catttcggag tgtatactcg cgtgtcgcag      1260
   tacattgaat ggttgcagaa actcatgcgc tccgagcctc ggccgggagt gctgctgaga      1320
   gccccctttc cg                                                           1332
<210> 4
   <211> 1563
   <212> DNA
   <213> Arthrobacter ureafaciens
<400> 4
   atggcaactg gttcgcggac ctccttgctt ctcgccttcg gcctgctctg tctgccctgg      60
   ttgcaagagg gaagcgctgc ccctacccct ccgaacagcc cgactctccc gccaggatcc     120
   ttcagcgaaa ctaatctggc tgcagatcgg actgccgcca actttttcta ccgcatccca     180
   gccctgacct acttgggcaa cgacgtggtg ctggccgcat gggacggacg gcctggatca     240
   gcggccgatg ctccaaaccc gaactcgatc gtccagaggc gctccaccga tggcggaaaa     300
   acttggggcc cagtgcaagt gatcgctgct ggccatgtcg ccgacgcgtc cggaccgcgg     360
   tacggatact cagacccctc gtacatctac gacgcagaag cgaacaaggt gttcgccttc     420
   ttcgtctact ccaaggatca aggctttggc ggctcccagt tcggaaatga cgatgccgat     480
   cgcaacgtga tttcgtcggc cgtgatcgaa agctcggacg cgggagtgac ctggtcccag     540
   cctcggctca tcacctcggt gactaagccg ggtacgtcaa agaccaatcc ggcagcaggg     600
   gatgtgagat ccaatttcgc ctcatcgggc gaggggattc agctcaagta cggaccgcat     660
   aagggtcgcc tgatccaaca gtacgcggga gatgtcagac aggctgacgg atcaaacaaa     720
   atccaagcgt attccgtgta ctcggacgac cacggcgtga cctggcacaa aggtgccaat     780
   gtcggagata ggatggacga aaacaagacc gtggaactga gcgacggacg ggtcctgctt     840
   aattcgcgcg acaacgctaa tcgcggctat agaaaggtgg cggtgtcgac tgatggaggg     900
   gcaacttacg gaccggtcag ccaggacacc gagctgccag acccggcgaa caatggtgca     960
   attgcgcgca tgttcccgaa tgccgcgcag gggtccgcgg atgcgaaaaa gctgatcttc    1020
   accaacgcca attcaaagac tggacgggaa aacgtgtccg ctcgcgtgtc atgcgatgac    1080
   ggagagactt ggccgggagt gcgcaccatc aggagcggat tcagcgcata ctccaccgtg    1140
   acccggctgg ccgacggaaa gttcggagtc ctctacgagg ggaattacac cgataacatg    1200
   ccatttgcca cttttgatga cgcttggttg aactacgtgt gcgctcctct cgcggtccct    1260
   gctgtgaaca tcgccccgtc cgcaacccaa gaagtgccag tcactgtcac caaccaggag    1320
   gccacgaccc tttcgggagc aaccgctact gtgtacaccc cgagcggctg gtcggccact    1380
   actgtcccgg tgcctgacgt ggcccctggt gcctccgtga cggtgacggt cgcgctgacc    1440
   gcaccagcgg acgcctcggg cccaagaagc ctgaacgccg ccttcacgac cgccgatggt    1500
   agagtgtcac agtttacctt tactgcaact actcccgtcg ctccccaagt gggactgacc    1560
   atc                                                                 1563
<210> 5
   <211> 521
   <212> PRT
   <213> Arthrobacter ureafaciens
<400> 5
   Met Ala Thr Gly Ser Arg Thr Ser Leu Leu Leu Ala Phe Gly Leu Leu
   1               5                   10                  15
   Cys Leu Pro Trp Leu Gln Glu Gly Ser Ala Ala Pro Thr Pro Pro Asn
               20                  25                  30
   Ser Pro Thr Leu Pro Pro Gly Ser Phe Ser Glu Thr Asn Leu Ala Ala
           35                  40                  45
   Asp Arg Thr Ala Ala Asn Phe Phe Tyr Arg Ile Pro Ala Leu Thr Tyr
       50                  55                  60
   Leu Gly Asn Asp Val Val Leu Ala Ala Trp Asp Gly Arg Pro Gly Ser
   65                  70                  75                  80
   Ala Ala Asp Ala Pro Asn Pro Asn Ser Ile Val Gln Arg Arg Ser Thr
                   85                  90                  95
   Asp Gly Gly Lys Thr Trp Gly Pro Val Gln Val Ile Ala Ala Gly His
               100                 105                 110
   Val Ala Asp Ala Ser Gly Pro Arg Tyr Gly Tyr Ser Asp Pro Ser Tyr
           115                 120                 125
   Ile Tyr Asp Ala Glu Ala Asn Lys Val Phe Ala Phe Phe Val Tyr Ser
       130                 135                 140
   Lys Asp Gln Gly Phe Gly Gly Ser Gln Phe Gly Asn Asp Asp Ala Asp
   145                 150                 155                 160
   Arg Asn Val Ile Ser Ser Ala Val Ile Glu Ser Ser Asp Ala Gly Val
                   165                 170                 175
   Thr Trp Ser Gln Pro Arg Leu Ile Thr Ser Val Thr Lys Pro Gly Thr
               180                 185                 190
   Ser Lys Thr Asn Pro Ala Ala Gly Asp Val Arg Ser Asn Phe Ala Ser
           195                 200                 205
   Ser Gly Glu Gly Ile Gln Leu Lys Tyr Gly Pro His Lys Gly Arg Leu
       210                 215                 220
   Ile Gln Gln Tyr Ala Gly Asp Val Arg Gln Ala Asp Gly Ser Asn Lys
   225                 230                 235                 240
   Ile Gln Ala Tyr Ser Val Tyr Ser Asp Asp His Gly Val Thr Trp His
                   245                 250                 255
   Lys Gly Ala Asn Val Gly Asp Arg Met Asp Glu Asn Lys Thr Val Glu
               260                 265                 270
   Leu Ser Asp Gly Arg Val Leu Leu Asn Ser Arg Asp Asn Ala Asn Arg
           275                 280                 285
   Gly Tyr Arg Lys Val Ala Val Ser Thr Asp Gly Gly Ala Thr Tyr Gly
       290                 295                 300
   Pro Val Ser Gln Asp Thr Glu Leu Pro Asp Pro Ala Asn Asn Gly Ala
   305                 310                 315                 320
   Ile Ala Arg Met Phe Pro Asn Ala Ala Gln Gly Ser Ala Asp Ala Lys
                   325                 330                 335
   Lys Leu Ile Phe Thr Asn Ala Asn Ser Lys Thr Gly Arg Glu Asn Val
               340                 345                 350
   Ser Ala Arg Val Ser Cys Asp Asp Gly Glu Thr Trp Pro Gly Val Arg
           355                 360                 365
   Thr Ile Arg Ser Gly Phe Ser Ala Tyr Ser Thr Val Thr Arg Leu Ala
       370                 375                 380
   Asp Gly Lys Phe Gly Val Leu Tyr Glu Gly Asn Tyr Thr Asp Asn Met
   385                 390                 395                 400
   Pro Phe Ala Thr Phe Asp Asp Ala Trp Leu Asn Tyr Val Cys Ala Pro
                   405                 410                 415
   Leu Ala Val Pro Ala Val Asn Ile Ala Pro Ser Ala Thr Gln Glu Val
               420                 425                 430
   Pro Val Thr Val Thr Asn Gln Glu Ala Thr Thr Leu Ser Gly Ala Thr
           435                 440                 445
   Ala Thr Val Tyr Thr Pro Ser Gly Trp Ser Ala Thr Thr Val Pro Val
       450                 455                 460
   Pro Asp Val Ala Pro Gly Ala Ser Val Thr Val Thr Val Ala Leu Thr
   465                 470                 475                 480
   Ala Pro Ala Asp Ala Ser Gly Pro Arg Ser Leu Asn Ala Ala Phe Thr
                   485                 490                 495
   Thr Ala Asp Gly Arg Val Ser Gln Phe Thr Phe Thr Ala Thr Thr Pro
               500                 505                 510
   Val Ala Pro Gln Val Gly Leu Thr Ile
           515                 520